**Europäisches Patentamt**

**European Patent Office**

**Office européen des brevets**

(19)

(11) Publication number: **0 181 161**

**A2**

(12)

# EUROPEAN PATENT APPLICATION

(21) Application number: **85307924.2**

(22) Date of filing: **31.10.85**

(51) Int. Cl.⁴: **A 61 K 7/22**
**A 61 K 7/24**

(30) Priority: **31.10.84 GB 8427499**

(43) Date of publication of application:
**14.05.86 Bulletin 86/20**

(84) Designated Contracting States:
**BE CH DE FR GB IT LI NL SE**

(71) Applicant: **Stafford-Miller Ltd.**
**The Common 32-36**
**Hatfield Hertfordshire AL10 0NZ(GB)**

(84) Designated Contracting States:
**CH GB LI SE**

(71) Applicant: **STAFFORD-MILLER CONTINENTAL, NV-SA**
**Nijverheidsstraat, 22**
**Oevel, Belgium(BE)**

(84) Designated Contracting  es:
**BE DE FR IT NL**

(72) Inventor: **George, Dienc**
**'Summerfields' Brantingham**
**Elloughton Nr. Humberside(GB)**

(72) Inventor: **Modley, John Martin**
**13 The Drive**
**Hartley Plymouth(GB)**

(74) Representative: **Ablewhite, Alan James et al,**
**MARKS & CLERK 57/60 Lincoln's Inn Fields**
**London WC2A 3LS(GB)**

(54) Oral hygiene products.

(57) A fluoride-free oral hygiene product contains in solution a cationic bis-biguamide antiseptic such as chlorohexidine and zinc ions, the zinc ions being provided by a soluble zinc salt of an aldonic acid such as gluconic acid. The antiseptic is preferably also in the form of a soluble salt with an aldonic acid.

## ORAL HYGIENE PRODUCTS.

This invention relates to oral hygiene products containing zinc, particularly for use in the treatment of dental and periodontal disorders.

The use of zinc in oral compositions such as mouthwashes and toothpastes has been widely reported. Formulations can be divided into those containing sparingly soluble zinc compounds and those containing zinc ions in solution. In the former category may be included dentifrice compositions such as those disclosed in British Patent Specifications 1373001 and 1373003 of Unilever Limited. Typical zinc salts are those with various carboxylic acids such as zinc benzoate, zinc caproate and zinc citrate and other salts such as alkylbenzylacrylate salts, hexafluorosilicate salts and soaps with various long chain fatty acids.

In the latter type of composition the zinc is present in solution and is provided by a soluble zinc salt. For example, British Patent Application 2084870A of H.R.Muhlemann describes a combination of zinc salts such as zinc fluoride, zinc acetate and zinc citrate with an antibacterial pyrimidineamine base, especially hexetidine. A dentifrice is also known containing zinc phenylsulphonate. There are many publications of compositions based on zinc chloride. For example British Patent 2014851 of Johnson & Johnson discloses compositions containing zinc chloride in combination with glycerol, while U.K. Patent Application 2052978A of Unilever Limited discloses compositions containing zinc chloride together with glycine. U.K.Patent Application 2080681A of Fahim & Miller discloses compositions based on a combination of a soluble salt with ascorbic acid. U.S.Patent Specification 4082841 of Pader assigned to Lever Brothers Company discloses the combination of zinc ions with an organoleptically acceptable enzyme, particularly a protease, carbohydrase or lipase.

Of particular interest in dental application is the combination of a soluble zinc salt such as zinc chloride with a cationic bis-biguanide antiseptic buffered at near neutrality. A typical formulation contains zinc chloride and chlorhexidine gluconate buffered at pH 6.5 with a sodium acetate/acetic acid buffer system. However, we find that with this kind of formulation lack of stability can be a problem. The chlorhexidine tends to degrade so that prolonged storage in warm conditions leads to significant loss in the available chlorhexidine.

We have now found that a formulation of this type can be markedly improved in stability if the zinc is provided as a zinc salt of an aldonic acid such as gluconic acid and especially if the chlorhexidine and the zinc share a common anion.

US 4,469,674 assigned to Richardson-Vicks Inc discloses the use of zinc salts, inter alia zinc gluconate, in the presence of fluoride ions in oral compositions. These fluoride and zinc formulations can also contain an antibacterial agent and many types are listed, including chlorhexidine and acid addition salts thereof. There is no mention of any salts of aldonic acids in this context.

According to the present invention there is provided a fluoride-free oral hygiene product containing in solution a cationic bis-biguanide antiseptic and zinc ions, characterised in that the zinc ions are provided by a soluble zinc salt of an aldonic acid, especially gluconic acid, and preferably in that the bis-biguanide antiseptic is also provided as a soluble salt with an aldonic acid.

The cationic bis-biguanide may be any one of the group of compounds of this type used as antiseptics, for example alexidine and especially chlorhexidine. The aldonic acid is conveniently gluconic acid, the gluconate being a commonly used salt for bis-biguanides of this type. Obviously, other aldonic acids could be substituted, e.g. the galactonic or mannonic acids.

The oral hygiene product according to the invention may comprise a mouthwash type of formulation, especially for use against gingivitis, or a dentifrice or toothpaste formulation, especially for use against plaque and calculus. Other formulations are possible, e.g. lozenges and paints.

The concentration of the bis-biguanide salt in the formulation may typically be from 0.025 to 0.2% by weight, especially about 0.05 to 0.15%. The concentration of zinc ion may be from 0.05 to 4% especially about 0.1 to 0.15% by weight.

The preferred buffer system for use according to the present invention is based on the lactone of the aldonic acid, for example gluconolactone, and an alkali such as sodium hydroxide.

The following examples illustrate the invention further:

## Example 1

### Mouthwash formulation

| Ingredients | Concentration % w/w |
|---|---|
| Chlorhexidine gluconate (20% solution) | 0.5647 |
| Zinc gluconate | 0.84 |
| Sodium Saccharin | 0.015 |
| Gluconolactone | 1.2 |
| Sodium Hydroxide | 0.27 |
| Patent Blue V | 0.00125 |
| Quinoline yellow | 0.00075 |
| Glycerin | 10.0 |
| Tween 80 | 1.0 |
| Alcohol (absolute) | 15.0 |
| Flavour Mix (Mint/eugenol) to | 0.030775 |
| Deionized water to | 100.0 |

## Example 2

### Mouthwash formulation

| Ingredients | Concentration % w/w |
|---|---|
| Chlorhexidine gluconate (20% solution) | 0.5591 |
| Zinc gluconate | 0.84 |

| | |
|---|---|
| Sodium Saccharin | 0.0075 |
| Gluconolactone | 1.2 |
| Sodium Hydroxide | 0.27 |
| Patent Blue V | 0.00125 |
| Quinoline yellow | 0.00075 |
| Glycerin | 10.0 |
| Tween 80 | 1.0 |
| Alcohol (absolute) | 20.00 |
| Flavour Mix (Spearmint) | 0.0545 |
| Deionized water to | 100.0 |

The flavour can be any typical mouthwash flavour, e.g a mint or spearmint formulation, a lemon formulation or a so-called eugenol formulation. The formulation may optionally also contain an analgesic such as tetracaine at 0.5%.

Stability tests were carried out using formulations as described above in comparison with formulations in which the zinc gluconate was replaced by zinc chloride (0.25%) and the gluconolactone/sodium hydroxide buffer by sodium acetate (1.64%) and glacial acetic acid (0.0063%). Samples were stored for 6 weeks at 40°C and tested for the loss in chlorhexidine. In a mint flavoured composition, the chlorhexidine loss was 4.1% as opposed to 19% in the zinc chloride based comparison formulation, while in a eugenol flavoured composition, the chlorhexidine loss was 1.8% compared with 11% in the comparison formulation.


Example 3


Toothpaste | % (w)
--- | ---
Sorbitol | 40
Water | 40
chlorohexidine gluconate | 0.05
Zinc gluconate | 0.8
Polyethyleneglycol | 5
Silicate | 10
Nipagin | 0.2
Nipasol | 0.1
Sodium monofluorophosphate | 0.8
Flavouring etc    to | 100


Example 4


Lozenge | % (w)
--- | ---
chlorohexidene gluconate | 0.05
zinc gluconate | 0.8
lozenge base, e.g. gelatin and colouring and flavours, to | 100

CLAIMS:

1. A fluoride-free oral hygiene product containing in solution a cationic bis-biguanide antiseptic and zinc ions, characterised in that the zinc ions are provided by a soluble zinc salt of an aldonic acid.

2. An oral hygiene product according to claim 1, in which the bis-biguanide is provided as a soluble salt with an aldonic acid.

3. An oral hygiene product according to claim 1 or claim 2, in which the aldonic acid is gluconic acid.

4. An oral hygiene product according to any of claims 1 to 3, in which the bis-biguanide antiseptic is chlorhexidine.

5. An oral hygiene product according to any of claims 1 to 4, in which the concentration of bis-biguanide salt is from 0.025 to 0.2% by weight.

6. An oral hygiene product according to claim 5, in which the concentration of bis-biguanide salt is from 0.05 to 0.15% by weight.

7. An oral hygiene product according to any of claims 1 to 6, in which the concentration of zinc ion is from 0.05 to 4% by weight.

8. An oral hygiene product according to claim 7, in which the concentration of zinc ion is from 0.1 to 0.15% by weight.

9. An oral hygiene product according to any of claims 1 to 8 containing a buffer system based on the lactone of the aldonic acid and an alkali.

10. An oral hygiene product according to any of claims 1 to 9 in the form of a mouthwash, toothpaste or dentifrice.